# EUROPÄISCHE PATENTANMELDUNG

(11) **EP 2 801 341 A1**
(43) Veröffentlichungstag der Anmeldung: **12.11.2014**
(21) Anmeldenummer: 14156689.3
(22) Anmeldetag: 26.02.2014
(51) Int. Cl.: A61F 2/966

(54) **Freigabevorrichtung zum Lösen eines medizinischen Implantats von einer Einführvorrichtung sowie Einführvorrichtung mit einer Freigabevorrichtung**

(30) Priorität: 06.05.2013 US 201361819674 P
(71) Anmelder: Biotronik AG, 8180 Bülach (CH)
(72) Erfinder: Quint, Bodo, 8154 Oberglatt (CH)
(74) Vertreter: Lindner-Vogt, Karin L.

(57) **Zusammenfassung**

Die Erfindung betrifft Freigabevorrichtung (100, 100a) zum Lösen eines medizinisches Implantats (105) von einer Einführvorrichtung (110), bei welcher das Implantat (105) durch eine Relativbewegung zwischen einem ersten und einem zweiten Einführelement (92, 94) freisetzbar ist, umfassend einen Körper (10, 10a) mit einem proximalen Ende (12), mit einem proximalen Ende (12), das im Benutzungszustand von einem distalen Ende (120) der Einführvorrichtung (110) entfernt ist, und einem distalen Ende (14), das im Benutzungszustand dem distalen Ende (120) der Einführvorrichtung (110) zugewandt ist, wobei zwischen dem proximalen und dem distalen Ende (12, 14) eine Handgriffeinheit (16) mit einem relativ zum Körper (10, 10a) fixierten ersten Griffsegment (18) und zumindest einem beweglich angeordneten zweiten Griffsegment (20, 20a) vorgesehen ist, wobei das beweglich angeordnete zweite Griffsegment (20, 20a) in Umfangsrichtung (22) drehbar ist, um einer gezielte Relativbewegung zwischen dem ersten und dem zweiten Einführelement (92, 94) der Einführvorrichtung (110) zu bewirken.

Die Erfindung betrifft ferner eine Einführvorrichtung (110) mit einer solchen Freigabevorrichtung (100, 100a).

## Beschreibung

Die Erfindung betrifft eine Freigabevorrichtung zum Lösen eines medizinischen Implantats von einem Katheter und einen Katheter mit einer Freigabevorrichtung zum Freigeben eines medizinischen Implantats zur Implantation in einen tierischen und/oder menschlichen Körper nach den Oberbegriffen der unabhängigen Patentansprüche.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Himschrittmacher für Parkinsonpatienten, Herzimplantate, wie Herzklappen oder so genannte Septum-closure devices, Cochleaimplantate, Retina Implantate, zahnmedizinische Implantate, Implantate zum Gelenkersatz, Gefäßprotesen z. B. einsetzbar in die Pulmunarvene, Okkluder, beispielsweise für den Appendix, oder Stents.

Implantate sind zum Einführen in den Körper mit Kathetern verbunden und müssen am Einsatzort genau platziert und definiert freigegeben werden können. Hierzu ist beispielsweise bekannt, das Implantat durch eine Schiebbewegung freizugeben.

Eine besondere Herausforderung ist es große Implantate, die in der Regel auch ein steiferes Liefersystem benötigen, in einer stark zeitlich veränderlichen Anatomie freizusetzen. Hier kann durch Gefäßkontakt und zu langsames Freisetzen des Implantats ein Trauma entstehen oder das Implantat kann durch die veränderliche Anatomie in seiner beabsichtigten Platzierung versetzt werden. Dies gilt im Besonderen für minimal invasiv implantierbare Herzklappen, so genannte Appendix closure devices und für die Platzierung von Nitinol Stents in den Pulmunarvenen.

Der Erfindung liegt die Aufgabe zugrunde, eine Freigabevorrichtung anzugeben, mit der das gezielte Freigeben eines Implantats verbessert ist.

Eine weitere Aufgabe ist in der Bereitstellung einer entsprechenden Einführvorrichtung zu sehen.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Es wird eine Freigabevorrichtung zum Lösen eines medizinischen Implantats von einer Einführvorrichtung vorgeschlagen, bei welcher das Implantat durch eine Relativbewegung zwischen einem ersten und einem zweiten Einführelement freisetzbar ist. Die Freigabevorrichtung umfasst einen Körper, mit einem proximalen Ende, das im Benutzungszustand von einem distalen Ende der Einführvorrichtung entfernt ist, und einem distalen Ende, das im Benutzungszustand dem distalen Ende der Einführvorrichtung zugewandt ist, wobei zwischen dem proximalen und dem distalen Ende eine Handgriffeinheit mit einem relativ zum Körper fixierten ersten Griffsegment und zumindest einem beweglich angeordneten zweiten Griffsegment vorgesehen ist, wobei das beweglich angeordnete zweite Griffsegment in Umfangsrichtung drehbar ist, um einer gezielte Relativbewegung zwischen dem ersten und dem zweiten Einführelement der Einführvorrichtung zu bewirken.

Durch die erfindungsgemäße Ausgestaltung kann eine Freigabevorrichtung bereitgestellt werden, die intuitiv bedienbar gestaltet ist. Ferner kann das drehbare zweite Griffsegment bzw. der Drehmechanismus mit einer ganzen Hand umgriffen werden. Zudem beinhaltet es mindestens zwei Funktionen. Durch Drehung des zweiten Griffsegments wird eine langsame, dosierte Freigabe des Implantats gesteuert und durch Zug an dem zweiten Griffsegment erfolgt die unmittelbare und schnelle Freisetzung. Dies ist besonders bedienerfreundlich, da es für den Bediener, insbesondere durch das Umgreifen mit zwei Händen, erfolgen kann. Des Weiteren kann auch die fixierende Hand, insbesondere angreifend am ersten Griffsegment, stets ihre Funktion der Lagebestimmung eines Liefersystems, wie einem Katheter, relativ zu dem behandelten Patienten beibehalten werden. Da die ganzen Hände genutzt werden können und Funktionen nicht auf einzelne Finger in der Bedienung begrenzt werden, können manuell große Kräfte eingeleitet werden. Ferner können mittels der Drehbewegung untersetzte, kraftvolle Bewegungen übertragen werden, durch die das Implantat zuverlässig und homogen freigegeben bzw. präzise und vorsichtig positioniert werden kann. Somit kann sowohl eine kraftvolle und /oder eine fein dosierte, vorsichtige Freisetzung ermöglicht werden. Zudem wird so ein einfaches, in der Anwendung auch schnell umsetzbares Konzept zu einer Freigabe des Implantats verwirklicht. Besonders vorteilhaft kann mittels der erfindungsgemäßen Ausgestaltungen eine Repositionierung oder ein Rückzug des teilweise freigegebenen Implantats, beispielsweise bei Fehlpositionierung, ermöglicht werden. Dies ist insbesondere der Fall, da die Drehbewegung eine große Kraft zum wieder Zusammendrücken des teilweise expandierten Implantats erzeugen kann. Des Weiteren weist die Freigabevorrichtung eine einfache Handhabung und Montage des Implantats auf der Einführvorrichtung, beispielsweise einem Katheter, im Vorbereitungslabor auf. Das erfindungsgemäße Konzept sich insbesondere gut und vorteilhaft einsetzbar für eine bewegliche Anatomie, wie beispielsweise am Herzen.

In diesem Zusammenhang soll unter einer "Griffeinheit" eine Einheit verstanden werden, die von einem Bediener, wie einem Monteur, einem Arzt oder einer Pflegeperson, mit zumindest einer Hand, angefasst, umgriffen und/oder bedient wird, insbesondere bei einer Montage des Implantats im Labor oder einer Freigabe des Implantats bei der Implantation im Körper. Unter eine "Griffsegment" soll ein Anteil oder Bereich der Griffeinheit verstanden werden, der sich in zumindest einem Parameter, wie einer Platzierung, einer Funktion, einem Bedienmechanismus und/oder einem anderen vom Fachmann für sinnvoll erachteten Parameter, von zumindest einem anderen Anteil oder Bereich der Handgriffeinheit unterscheidet. Bevorzugt erstreckt sich das zweite Griffsegment in Umfangsrichtung zu zumindest 50%, vorteilhaft zu zumindest 75% und besonders bevorzugt zu zumindest 100% um einen Umfang des Körpers. Das erste Griffsegment ist vorteilhafterweise relativ zum Körper bzw. den Bauteilen im Inneren des Körpers fixiert angeordnet und/oder ist einstückig mit dem Körper bzw. dessen Gehäuse ausgebildet, wobei unter "einstückig" verstanden werden soll, dass das erste Griffelement und der Körper von demselben Bauteil gebildet sind und/oder nur unter Funktionsverlust zumindest eines der Bauteile voneinander getrennt werden können. Ferner ist es distal vom zweiten Griffsegment angeordnet. Unter dem Begriff "bewirken" soll hier und im weiteren Text "erzeugen, veranlassen und/oder erreichen" verstanden werden.

Vorteilhafterweise vermittelt das zweite Griffsegment die Relativbewegung zwischen dem ersten und dem zweiten Einführelement über zumindest einen Wirkschluss mit einem Gewinde. Die durch die Drehbewegung des zweiten Griffsegments ergonomisch hohen und einfach realisierbaren Kräfte, können durch den Einsatz des Gewindes besonders gleichmäßig übertragen werden. Zudem ist eine präzise und feinfühlige Manipulation möglich und dies sogar trotz eines möglichen Einsatzes einer hohen Gangsteigung der "Gewindeübertragung" für die Untersetzung. In diesem Zusammenhang soll unter einem Wirkschluss insbesondere ein Formschluss und/oder ein Kraftschluss verstanden werden. Unter einem Gewinde soll in diesem Zusammenhang ein Wirkelement verstanden werden, dass die Wirkung des zweiten Griffsegments vermittelt und/oder überträgt. Es soll darunter insbesondere auch eine Spindel oder eine Schnecke verstanden werden. Das Gewinde ist bevorzugt relativ zu dem ersten Griffsegment fixiert bzw. in/an einem Bauteil, wie einem Grundkörper, angeordnet oder ausgebildet, das relativ zu dem ersten Griffsegment fixiert angeordnet ist. Zudem ist das Gewinde relativ zu einem Einführelement und insbesondere einem inneren Einführelement und beispielsweise im Falle eines Katheters relativ zum Innenschaft fixiert.

Des Weiteren wird vorgeschlagen, dass der Wirkschluss zwischen dem zweiten Griffsegment und dem Gewinde über zumindest ein Koppelelement erfolgt, wodurch die Übertragung variierbar gestaltet werden kann. Das Koppelelement kann von jedem, dem Fachmann für einsetzbar erachteten Element gebildet sein, wie einem Stift, einer Walze oder bevorzugt einer Kugel. Zudem ist das Koppelelement in einer vorteilhaften Realisierung als ein mitlaufendes, insbesondere axial mitlaufendes, Koppelelement ausgeführt. Vorteilhafterweise ist das Koppelelement in einem Käfig, insbesondere einem axial bewegbaren Käfig, gehalten. Ist das Koppelelement beispielsweise von einer Kugel gebildet, ist der Käfig als ein Kugelkäfig ausgebildet. Ferner ist es vorteilhaft, wenn der Wirkschluss zwischen dem zweiten Griffsegment und dem Gewinde zumindest einen Kraftschluss darstellt, wodurch Kräfte konstruktiv einfach übertragen werden können. Alternativ und/oder zusätzlich wäre auch denkbar, dass der Wirkschluss einen Formschluss darstellt, wodurch eine stabile Wirkverbindung gestaltbar ist.

In einer alternativen Ausgestaltung der Erfindung wird vorgeschlagen, dass der Wirkschluss zwischen dem zweiten Griffsegment und dem Gewinde über zumindest ein Verzahnungselement erfolgt. Dadurch kann eine besonders zuverlässige und unanfällige Verbindung gewährleistet werden. Das Verzahnungselement kann von jedem, dem Fachmann für einsetzbar erachteten Element gebildet sein, wie einem Zahnrad, einer Spindel, einer Schnecke oder bevorzugt einer Zahnstange.

Ferner wird vorgeschlagen, dass die Relativbewegung zwischen dem ersten und dem zweiten Einführelement mittels eines Verbindungselements auf eines der Einführelemente übertragbar ist. Dadurch kann eine Übertragung besonders flexibel ausgestaltet werden. Ferner können Eigenschaften, wie eine Material, eine Materialhärte, eine Orientierung und/oder eine andere vom Fachmann für sinnvoll erachtete Eigenschaft, jedes Bauteils auf ihre individuelle Funktion ohne Beeinträchtigung von einer Funktion eines anderen Bauteils abgestimmt werden. Hierbei ist das Einführelement bevorzugt ein äußeres Einführelement bzw. beispielsweise im Fall eines Katheters ein Außenschaft. Des Weiteren kann es vorteilhaft sein, wenn das Verbindungselement wirkmäßig mit dem zweiten Griffsegment verbunden ist, wodurch die Übertragung der Bewegung zuverlässig erfolgen kann. In diesem Zusammenhang soll unter dem Begriff "wirkmäßig" verstanden werden, dass über die Verbindung von Verbindungselement und zweitem Griffsegment die Wirkung des zweiten Griffsegments vermittelbar oder übertragbar ist.

Zudem kann eine sichere Platzierung von Bauteilen, insbesondere auch relativ zueinander, vorteilhaft erreicht werden, wenn eine Fixierung des Gewindes bzw. des Bauteils mit Gewinde (der Grundkörper), mittels zumindest eines Fixierelements erfolgt. Hierbei kann das Fixierelement von jedem, dem Fachmann für einsetzbar erachteten Bauteil gebildet sein, wie beispielsweise einem Stift, einem Haken, einer Wand oder einer Platte. Zudem wird vorgeschlagen, dass sich das Fixierelement im Wesentlichen radial, bevorzugt radial, zu einer Innenachse der Einführvorrichtung erstreckt. Dadurch kann die Fixierung besonders Platz sparend gestaltet werden Unter radial soll hier senkrecht verstanden werden. Ferner soll unter "im Wesentlichen radial" eine Abweichung der Richtung des Fixierelements zu der Richtung der Innenachse von der radialen bzw. senkrechten Anordnung von bis zu 30° verstanden werden. Das Fixierelement ist vorteilhafterweise am ersten Griffsegment angeordnet, wodurch die ortsfeste Anordnung konstruktiv einfach realisierbar ist.

Vorteilhafterweise erstreckt sich das Fixierelement alternativ und/oder zusätzlich im Wesentlichen axial, bevorzugt axial, zu der Innenachse der Einführvorrichtung. Dadurch kann die Funktion ein axial verschiebbares Verbindungselement zu führen, unterstützt werden. Unter axial soll hier parallel zur Katheterachse verstanden werden. Ferner soll unter "im Wesentlichen axial" eine Abweichung der Richtung des Fixierelements zu der Richtung der Innenachse von der axialen bzw. parallelen Anordnung von bis zu 30° verstanden werden.

Eine bevorzugte Weiterbildung besteht darin, dass das Verbindungselement zumindest eine Ausnehmung aufweist, in die das zumindest eine Fixierelement eingreift. Hierdurch kann eine Position des Verbindungselements zuverlässig gesichert werden. Bevorzugt wird die Ausnehmung vom Fixierelement durchgriffen. Der Eingriff des Fixierelements in die bzw. durch die Ausnehmung dient insbesondere dazu, das Verbindungselement in Umfangsrichtung zu fixieren. Somit findet insbesondere keine Übertragung der Drehbewegung des zweiten Griffsegments auf das Verbindungselement und damit auf das Einführelement statt. Hierdurch werden schädigende Spannungen und Verdrillungen des Einführelements vermieden. Zudem kann das Fixierelement, insbesondere aufgrund seiner axialen Erstreckung, als ein, insbesondere axiales, Führungsmittel der Ausnehmung des Verbindungselements bei einer axialen Bewegung des Verbindungselements während der Relativbewegung zwischen dem ersten und dem zweiten Einführelement der Einführvorrichtung, beispielsweise bei der Freigabe des Implantats, dienen, wodurch diese Relativbewegung störungsfrei erfolgen kann.

In einer bevorzugten Realisierung ist das Verbindungselement mit einem Übertragungselement für die wirkmäßige Verbindung mit dem zweiten Griffsegment gekoppelt und/oder verbunden. Hierdurch können die Elemente und deren unterschiedliche Funktionen weiter entkoppelt werden. Das Übertragungselement weist zumindest den Käfig für das Koppelelement auf. Vorzugsweise ist das Übertragungselement über einen Formschluss mit dem Verbindungselement verbunden und/oder es greift über einen Fortsatz, der radial nach innen weist und sich in Umfangsrichtung erstreckt in eine Nut des Verbindungselements, die radial nach außen offen ist und sich ebenfalls in Umfangsrichtung erstreckt, ein. Damit ist ein Verdrehen der beiden Bauteile zueinander möglich bzw. das Übertragungselement ist relativ zum Verbindungselement drehbar angeordnet. Das Übertragungselement kann mit dem zweiten Griffsegment gedreht werden, ein Verdrehen des Verbindungselementes wird jedoch durch das Fixierelement verhindert.

Es wird zudem vorgeschlagen, dass das zweite Griffsegment in seinem axialen Ruhezustand durch zumindest ein Federelement vorgespannt ist, wodurch das zweite Griffsegment sicher in Position gehalten werden kann. Hierbei soll unter einem axialen Ruhezustand eine feste Position in axialer Richtung verstanden werden. Unter einem Federelement soll hier jedes federnde und/oder elastische Element, und insbesondere eine Feder, beispielsweise in der Form einer Druckfeder, verstanden werden. Zur Realisierung dieser erfindungsgemäßen Ausgestaltung weist das zweite Griffsegment an seiner Innenfläche eine Aussparung auf, in die ein radial nach außen weisender Fortsatz des Übertragungselements eingreift. Das Federelement ist axial zwischen den Fortsatz und einer Wandung der Aussparung des zweiten Griffsegments eingepasst.

Gemäß einer vorteilhaften Ausgestaltung ist der Wirkschluss zwischen dem zweiten Griffsegment und dem Gewinde durch eine Bewegung zumindest eines Entriegelungselement auflösbar. Hierdurch kann konstruktiv einfach ein weiterer Betriebsmodus der Freigabevorrichtung realisiert werden. Ein Entriegelungselement kann hier von jedem, dem Fachmann für anwendbar erachteten Bauteil gebildet sein, wie beispielsweise einem Drehelement, einem Kippelement und bevorzugt einem Schiebeund/oder Ziehelement. Bevorzugt ist das Entriegelungselement direkt am zweiten Griffsegment angeordnet, wodurch ein Umschalten der Betriebsmodi einfach und schnell, beispielsweise mit derselben Hand, erfolgen kann. Ein händisches Umgreifen kann somit Prozess straffend und Zeit sparend entfallen. Vorteilhafterweise ist der Wirkschluss gegen eine Federspannung zumindest eines Federelements auflösbar, wodurch die Aktivierungskraft, die für das Umschalten zwischen verschiedenen Betriebsmodi bzw. zwischen der Drehbewegung des zweiten Griffsegments und der Druck und/oder Zugbewegung des Entriegelungselement benötigt wird, technisch dosierbar ist. Das Federelement kann beispielsweise das Federelement sein, das das zweite Griffelement in seiner axialen Position vorspannt oder ein weiteres Federelement, dass an jeder anderen, dem Fachmann für sinnvoll erachteten Stelle angeordnet sein kann, wie beispielsweise am Entriegelungselement selbst.

Vorteilhafterweise bewirkt das Auflösen des Wirkschlusses eine schnelle Freisetzung des Implantats, wodurch die Freigabe des Implantats vorteilhaft für einen Patienten zuverlässig und schnell erfolgen kann. Dies kann insbesondere bei großen Implantaten und hohen Freigabekräften sinnvoll und vorteilhaft sein. Mittels der Drehbewegungen des zweiten Griffsegments können untersetzte, kraftvolle Bewegungen übertragen werden, die bei der schnellen Freisetzung durch Druck- und/oder Zugbewegung direkt übertragbar sind. Eine solche erfindungsgemäße Ausgestaltung ermöglicht eine intuitive Bedienung. Hierdurch können intensive Unterweisungen des Bedieners Zeit sparend entfallen. Zudem kann der Bediener eine Entscheidung zwischen einer sensitiven Freisetzung mittels Drehen des zweiten Griffsegments und möglichst spontaner endgültiger Freisetzung mittels des Entriegelungselements unverzögerte treffen. Die schnelle Freisetzung ist somit direkt und ungehindert durch zusätzliche Bedienmaßnahmen initiierbar. Mittels der Druck- und/oder Zugbewegung des Entriegelungselements wird eine gleitende und sanfte Freigabe des Implantats ermöglicht. Zudem entfällt durch die Anordnung des Entriegelungselements am zweiten Griffsegment eine Zweihandbedienung bzw. ein Handwechsel beim Umschalten auf die schnelle Freigabe. Somit kann eine schädigende Positionsänderung des Implantats Implantationsprozess verbessernd verhindert werden.

Als essentieller Vorteil kann aber die möglichst spontane Freisetzbarkeit des Implantats in einer zeitlich kritischen Situation empfunden werden. Diese Entscheidung und Reaktion kann zum Beispiel für die Platzierung einer Herzklappe wichtig sein. Grund hierfür ist der Kontakt zu einer beweglichen Anatomie, welche, ab Erkennung der korrekten Positionierung des Implantats bis zum endgültigen Freisetzen, zu Trauma, aber auch zur Versetzung des Implantats führen kann.

Ferner wird vorgeschlagen, dass sich durch die Bewegung des zumindest einen Entriegelungselements das zumindest eine Koppelelement radial weg von einer Innenachse der Einführvorrichtung bewegt und dadurch außer Eingriff mit dem Gewinde kommt. Hierdurch kann die Entkopplung konstruktiv einfach und schnell erfolgen. Bei einer Konstruktion mit Koppelelement und Käfig kann hierbei das Koppelelement bevorzugt in eine Aufnahme ausweichen, die an der Innenseite des zweiten Griffsegments angeordnet und/oder ausgeformt ist. Bei der Konstruktion mit dem Verzahnungselement kann dieses beispielsweise unter Zug bzw. Druck aus dem Formschluss mit dem Gewinde entfernt werden.

Alternativ und oder zusätzlich kann es auch vorgesehen sein, dass, abhängig von der Freigabeposition, beispielsweise die Funktion der schnelle Freigabe, zumindest ein Teil oder der gesamte der Entriegelungsmechanismus und/oder das Entriegelungselement lokal geblockt werden können.

Bevorzugterweise ist das zumindest eine Entriegelungselement einstückig mit dem zweiten Griffsegment ausgebildet, wodurch Platz, Montageaufwand und Kosten gespart werden können. Hierbei soll unter der Wendung "einstückig" verstanden werden, dass das Entriegelungselement und das zweite Griffsegment von demselben Bauteil gebildet sind und/oder nur unter Funktionsverlust zumindest eines der Bauteile voneinander getrennt werden können.

In einer alternativen oder zusätzlichen Ausgestaltung kann das Entriegelungselement von einem unabhängig von dem zweiten Griffsegment ausgebildeten Bauteil gebildet sein. Bevorzugt weist es einen Ring auf, der axial verschieblich und insbesondere in Umfangsrichtung um das zweite Griffsegment angeordnet ist. Vorteilhafterweise ist das Entriegelungselement in einer Einbuchtung des zweiten Griffsegments angeordnet, wobei das Entriegelungselement Platz sparend anordenbar ist. Zudem können so das zweite Griffelement und damit das äußere Einführelement unmittelbar nach der Entriegelung mit derselben Hand, die das Entriegelungselement bedient hat, axial bewegt werden.

In einer weiteren Ausgestaltung der Erfindung wird vorgeschlagen, dass der Körper, insbesondere das Bauteil mit Gewinde (der Grundkörper), eine Durchführung für eines der Einführelemente aufweist. Dies erlaubt eine kompakte Anordnung, welche das durchgeführte Einführelement stabilisiert und schützt. Ist die Einführvorrichtung ein Katheter, so kann das betreffende Einführelement ein Innenschaft des Katheters sein.

Gemäß einer vorteilhaften Ausgestaltung kann das Implantat ein selbstexpandierendes Implantat sein, wodurch es sich bei der Freigabe selbsttätig öffnen kann. Durch das selbstexpandierende Implantat kann ein zusätzliches Expandiermittel entfallen. Dadurch können vorteilhaft Raum und Montageaufwand für dieses eingespart werden. Hierdurch kann auch die Einführvorrichtung weniger komplex gestaltet werden. Grundsätzlich wäre es jedoch auch möglich, ein ballonexpandierbares Implantat zu verwenden. Hierfür müsste die Einführvorrichtung jedoch entsprechend angepasst werden, was der Fachmann aufgrund seiner Fachkenntnis selbstständig löst.

Gemäß einem weiteren Aspekt der Erfindung wird eine Einführvorrichtung zum Einführen des medizinischen Implantats vorgeschlagen, welches durch eine Relativbewegung zwischen dem ersten und dem zweiten Einführelement freisetzbar ist, umfassend die Freigabevorrichtung zum Lösen des medizinisches Implantats, umfassend den Körper, mit dem proximalen Ende, das im Benutzungszustand von dem distalen Ende der Einführvorrichtung entfernt ist, und dem distalen Ende, das im Benutzungszustand dem distalen Ende der Einführvorrichtung zugewandt ist, wobei zwischen dem proximalen und dem distalen Ende eine Handgriffeinheit mit einem relativ zum Körper fixierten ersten Griffsegment und zumindest einem beweglich angeordneten zweiten Griffsegment vorgesehen ist, wobei das beweglich angeordnete zweite Griffsegment in Umfangsrichtung drehbar ist, um einer gezielte Relativbewegung zwischen dem ersten und dem zweiten Einführelement der Einführvorrichtung zu bewirken.

Durch die erfindungsgemäße Ausgestaltung kann eine Einführvorrichtung bereitgestellt werden, die intuitiv bedienbar ist. Mittels der Drehbewegung können untersetzte, kraftvolle Bewegungen übertragen werden, durch die das Implantat zuverlässig und homogen freigegeben bzw. präzise und vorsichtig positioniert werden kann. Besonders vorteilhaft kann mittels der erfindungsgemäßen Ausgestaltungen eine Repositionierung oder ein Rückzug des teilweise freigegebenen Implantats, beispielsweise bei Fehlpositionierung, ermöglicht werden. Dies ist insbesondere der Fall, da die Drehbewegung eine große Kraft zum wieder Zusammendrücken des teilweise expandierten Implantats erzeugen kann. Des Weiteren weist die Einführvorrichtung eine einfache Handhabung und Montage des Implantats im Vorbereitungslabor auf.

Die Erfindung ist nachfolgend beispielhaft, anhand von in Zeichnungen dargestellten Ausführungsbeispielen, näher erläutert. Es zeigen in schematischer Darstellung:
- Fig.1: einen Schnitt durch ein günstiges Ausführungsbeispiel einer Einführvorrichtung und einer Freigabevorrichtung mit einem teilweise freigegebenen Implantat;
- Fig. 2: einen Schnitt durch die Freigabevorrichtung der Fig. 1 in einer Detaildarstellung;
- Fig. 3: einen Schnitt durch die Freigabevorrichtung der Fig. 1 in einer weiteren Detaildarstellung;
- Fig. 4: einen Schnitt durch die Freigabevorrichtung der Fig. 1 während einem Betriebsmodus einer schnellen Freigabe des Implantats und
- Fig. 5: einen Schnitt durch eine alternative Freigabevorrichtung.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

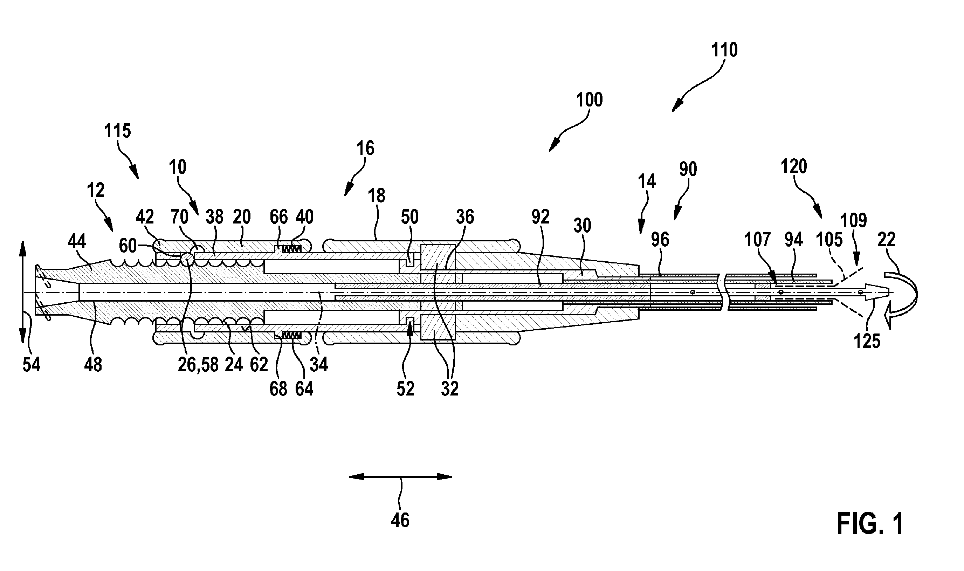

Fig. 1 zeigt einen Längsschnitt durch ein günstiges Ausführungsbeispiel einer Freigabevorrichtung 100 für ein medizinisches Implantat 105 einer nur teilweise dargestellten Einführvorrichtung 110. Die Einführvorrichtung 110 ist beispielsweise ein Katheter mit einem Schaftbereich 90 mit zwei koaxial angeordneten Einführelementen 92, 94, z.B. einem Innenschaft oder auch einem so genannten "pusher tube" (Einführelement 92) und einem diesen umgebenden Außenschaft oder einem so genannten "release tube" (Einführelement 94), welcher wiederum von einer Außenhülle 96 oder einem so genannten Stütztube umgeben sein kann. Die Außenhülle 96 hat die Funktion, die Einführelemente 92, 94 während der Einführung der Einführvorrichtung 110 in einen Patienten und bei einer Zuführung des Implantats 105 an einen Implantationsort zu stabilisieren. Hierbei hilft die Außenhülle 96 dem äußeren Einführelement 94 eine hemostatische Schleuse bzw. einen Introducer eines Einführungssystems zu passieren (nicht gezeigt). Anhand der Außenhülle 96 kann eine Freigabebewegung von dem Einführsystem entkoppelt und neutral durch das Einführungssystem geleitet werden, wodurch eine Relativbewegungen des Releasetubes bei einer Freigabe des Implantats 105 nicht auf den Schleusenbereich und die hemostatische Schleuse übertragen wird. Zudem kann so eine Reibung im Einführungssystem gemindert werden. Durch die Außenhülle 96 kann auch eine Kopplung zum Patienten entstehen, die nach Positionierung des Implantats 105 als Fixpunkt für die Position der Einführvorrichtung 110 dient. Dies kann zudem durch ein Schließen der hemostatischen Schleuse unterstützt werden. Das Pushertube (Einführelement 92) ist starr mit der Außenhülle 96 verbunden und verlängert den Schaftbereich 90 bis zu einer Katheterspritze 125. Ferner ist das innere Einführelement 92 mit der Katheterspitze 125 verbunden, das äußere Einführelement 94 hingegen nicht.

Da generell eine Reibung im Gefäß eher gering ist, ist es grundsätzlich möglich, die Außenhülle 96 in den Dimensionen nur auf den Schleusenbereich oder eine Bereich schlauchartiger Einführhilfen begrenzt auszuführen. Grundsätzlich, insbesondere wenn es für die Platzierung des Implantats nicht unbedingt nötig ist, kann die Freigabevorrichtung auch ohne Außenhülle ausgeführt sein. Üblicherweise ist dies beispielsweise bei Freigabevorrichtungen für Implantate, die auf Nitinolbasis gefertigt sind, der Fall. Hierbei wird die Reibung der Außenhülle 96, welche zu einem Versatz des Katheters führen kann, in Kauf genommen. Jedoch kann von einem dadurch erhaltenen verringerten Profil des Katheters profitiert werden. Von dem Anwender wird das Einführprofil des Katheters oft mit dem durch den minimalinvasiven Eingriff verbundenen Trauma für den Patienten in direkten Zusammenhang gesetzt.

Die Einführvorrichtung 110 ist in Anwenderbetrieb, also während einer Befestigung des Implantats 105 an der Freigabevorrichtung 100 oder während der Implantation mit ihrem proximalen Ende 115 einem Anwender zugewandt. Das Implantat 105 ist am distalen Ende 120 des Schaftbereichs 90 zwischen Innenschaft und Außenschaft platziert und soll am Implantationsort im tierischen oder menschlichen Körper freigegeben werden.

Die Freigabevorrichtung 100 dient zum Lösen des medizinischen Implantats 105 von der Einführvorrichtung 110. Das Implantat 105 ist an dem vom Anwender abgewandten Ende 120 des Schaftbereichs 90 beispielsweise in der Nähe der Katheterspitze 125 angeordnet. Das Implantat 105 ist beispielsweise um das innere Einführelement 92 gelegt und wird durch eine Relativbewegung zwischen dem ersten und dem zweiten Einführelement 92, 94 freigesetzt. Zur Freigabe wird das Releasetube (Einführelement 94) nach "proximal" bzw. in Richtung des proximalen Endes 115 gezogen und, vorausgesetzt ein distales Ende 109 des Implantats 105 ist außerhalb der Außenhülle 96 angeordnet, setzt somit das Implantat 105 frei.

Die Freigabevorrichtung 100 umfassend einen Körper 10 mit einem proximalen Ende 12, das im Benutzungszustand von dem distalen Ende 120 der Einführvorrichtung 110 entfernt ist, und mit einem distalen Ende 14, das im Benutzungszustand dem distalen Ende 120 der Einführvorrichtung 110 zugewandt ist. Zwischen dem proximalen und dem distalen Ende 12, 14 des Körpers 10 ist eine Handgriffeinheit 16 vorgesehen. Diese Handgriffeinheit 16 weist ein erstes Griffsegment 18 und ein zweites Griffsegment 20 auf. Das erste Griffsegment 18 ist relativ zum Körper 10 bzw. relativ zu Bauteilen, die im Inneren des Körpers 10 angeordnet sind, wie beispielsweise einem Grundkörper 44, einem Fixierelement 32 oder einem proximalen Bereich des inneren Einführelements 92, fixiert. Beispielsweise kann das erste Griffsegment 18 einstückig bzw. als ein Bauteil mit dem Körper 10 bzw. einem Gehäuse des Körpers 10 ausgebildet sein. Hierbei sind die Freigabevorrichtung 100 und die Einführvorrichtung 110 so ausgeführt, dass eine Lage des Körpers 10 und des ersten Griffsegments 18 relativ zum Implantat 105 und der Katheterspitze 125 veränderbar ist. Der Körper 10 ist über das erste Griffsegment 18 mit der Außenhülle 96 verbunden. Das zweite Griffsegment 20 erstreckt sich in Umfangsrichtung 22 komplett um einen Umfang des Körpers 10 und ist relativ zum Körper 10 und dem ersten Griffsegment 18 beweglich angeordnet. Zudem ist das erste Griffsegment 18 in Bezug auf das zweite Griffsegment 20 distal zu diesem bzw. in Richtung des distalen Endes 14 des Körpers 10 angeordnet. Das beweglich angeordnete zweite Griffsegment 20 ist in Umfangsrichtung 22 drehbar, wodurch die gezielte Relativbewegung zwischen dem ersten und dem zweiten Einführelement 92, 94 der Einführvorrichtung 110 bewirkt wird.

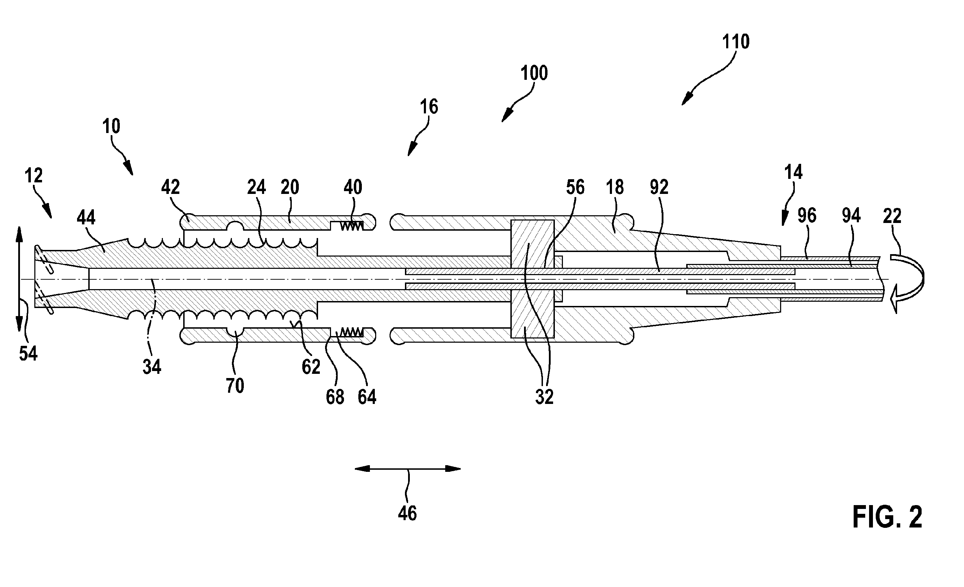

Wie auch im Detail in Fig. 2 zu sehen ist (in Fig. 2 bis 4 sind zur besseren Übersichtlichkeit nicht alle Bauteile eingezeichnet), weist der Körper 10 zudem einen Grundkörper 44 oder Katheterkörper auf, der sich in axialer Richtung 46 entlang einer Innenachse 34 der Einführvorrichtung 110 und durch jeweils das gesamte erste und zweite Griffsegment 18, 20 erstreckt. Der Grundkörper 44 ist mit dem inneren Einführelement 92 verbunden. Ferner weist der Körper 10 bzw. der Katheterkörper eine Durchführung 48 für das innere Einführelement 92 auf. Das innere Einführelement 92 bietet ein Lumen für beispielsweise einen nicht gezeigten Führungsdraht. Ferner ist am proximalen Ende 12 des Körpers 10 bzw. des Grundkörpers 44 ein Luer-Lock für eine Möglichkeit einer Spülung des inneren Einführelements 92 angebracht.

Zu einer Übertragung der Relativbewegung zwischen dem inneren und äußeren Einführelement 92, 94 weist der Körper 10 zudem eine Übertragungseinheit mit einem

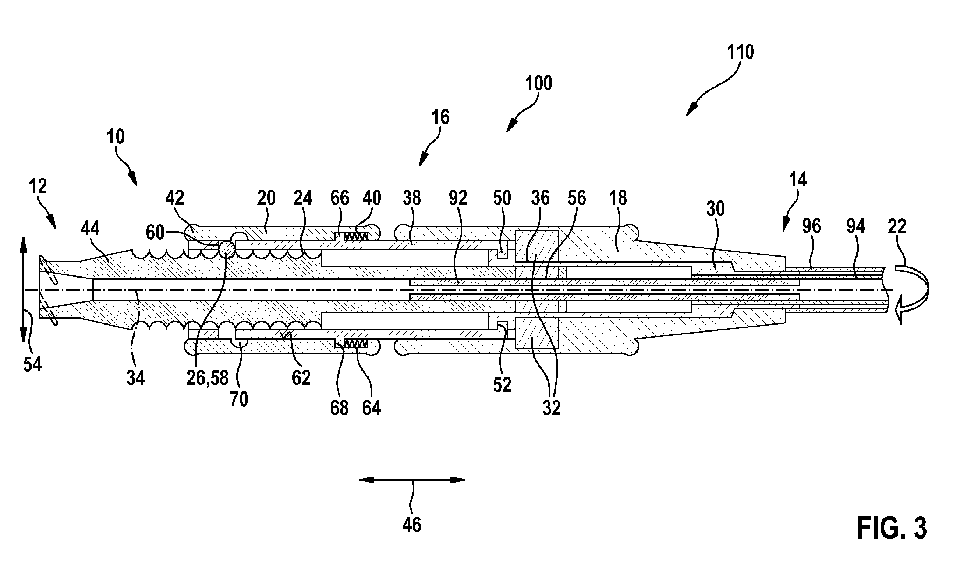

Übertragungselement 38 und einem Verbindungselement 30 auf. Das Übertragungselement 38 ist über einen Formschluss mit dem Verbindungselement 30 verbunden, wobei das Übertragungselement 38 relativ zum Verbindungselement 30 drehbar angeordnet ist (siehe unten). Hierfür weist das Übertragungselement 38 einen Fortsatz 50 auf, der radial nach innen zur Innenachse 34 weist und sich in Umfangsrichtung 22 erstreckt. Dieser Fortsatz 50 greift in eine Nut 52 des Verbindungselements 30, die für den Eingriff des Fortsatzes 50 radial nach außen offen ist und sich ebenfalls in Umfangsrichtung 22 erstreckt, ein. Das Übertragungselement 38 ist in radialer Richtung 54 radial zwischen dem zweiten Griffsegment 20 und dem Grundkörper 44 angeordnet, das Verbindungselement 30 hingegen in radialer Richtung 54 radial zwischen dem ersten Griffsegment 18 und dem Grundkörper 44. Am distalen Ende 14 des Körpers 10 ist das Verbindungselement 30 mit dem äußeren Einführelement 94 verbunden (vgl. Fig. 3).

Am ersten Griffsegment 18 sind zwei Fixierelemente 32, in Form von Platten, fixiert zu diesem angeordnet, wobei die zwei Fixierelemente 32 einander radial gegenüber angeordnet sind und sich zur Innenachse 34 bis zum Grundkörper 44 erstrecken. Hierfür weist das Verbindungselement 30 Ausnehmungen 36 auf, die in Umfangsrichtung 22 auf gleicher Höhe wie die Fixierelemente 32 angeordnet sind. In diese Ausnehmungen 36 greifen die Fixierelemente 32 ein bzw. diese werden von den Fixierelementen 32 durchgriffen. Hierdurch wird das Verbindungselement 30 in Umfangsrichtung 22 fixiert. Am Grundkörper 44 greifen die Fixierelemente 32 in Vertiefungen 56 ein und fixieren so den Grundkörper 44 sowohl axial wie auch in Umfangsrichtung 22 relativ zum ersten Griffsegment 18 (vgl. Fig. 2). Es entsteht somit eine starre, feste Kopplung zwischen dem ersten Griffsegment 18, mit den Fixierelementen 32 sowie der Außenhülle 96, und dem Grundkörper 44, mit dem inneren Einführelement 92.

Um die Drehung des zweiten Griffsegment 20 in eine Relativbewegung zwischen dem äußeren und dem inneren Einführelement 92, 94 bzw. eine axiale Bewegung umzuwandeln, weist der Grundkörper 44 im Bereich des zweiten Griffsegment 20 bzw. an seinem proximalen Ende 12 ein sich in Umfangsrichtung 22 erstreckendes Gewinde 24 auf. Der Grundkörper 44 verbindet somit funktionell das Gewinde 24 mit dem inneren Einführelement 92. Das zweite Griffsegment 20 vermittelt die Drehbewegung über einen Wirkschluss mit dm Gewinde 24, wobei der Wirkschluss einen Kraftschluss darstellt, der über ein Koppelelement 26 erfolgt. Das Koppelelement 26 ist von einer Kugel 58 gebildet, die in ihren Dimensionen auf die Dimensionen des Gewindes 24 abgestimmt ist. Zu einer Platzierung des Koppelelements 26 radial zwischen das zweite Griffsegment 20 und dem Grundkörper 44 bzw. dem Gewinde 24, weist das Übertragungselement 38 einen Käfig bzw. Kugelkäfig 60 in der Form einer Öffnung auf, in dem die Kugel 58 angeordnet ist.

Das Koppelelement 26 bzw. die Kugel 58 wird im Kugelkäfig 60 gehalten, da das zweite Griffsegment 20 in seinem axialen Ruhezustand durch zwei Federelemente 40, in der Form von Druckfedern, vorgespannt ist und somit das Koppelelement 26 radial abdeckt. Für eine Platzierung der Federelemente 40 weist des zweite Griffsegments 20 an einer Innenseite 62 pro Federelement 40 eine Aussparung 64 auf. Hierbei sind die Aussparungen 64 sich radial gegenüber angeordnet. Zu einer axial fixierten Ruhepositionierung an dem Übertragungselement 38 und zu einer verdrehungssicheren Kopplung zu dem Übertragungselement 38 sowie zu einer Übertragung der Drehbewegung des zweiten Griffsegments 20 auf das Übertragungselement 38 weist dieses zwei radial nach außen weisende Fortsätze 66 auf, von denen je einer in jeweils eine der Aussparungen 64 des zweiten Griffsegments 20 eingreift. Die Federelemente 40 sind nun jeweils axial zwischen den Fortsatz 66 und einer Wandung 68 der Aussparung 64 des zweiten Griffsegments 20 eingespannt.

Im Folgenden wird der Mechanismus der Freigabevorrichtung 100 für die Relativbewegung zwischen dem inneren und äußeren Einführelement 92, 94 durch Drehen des zweiten Griffsegments 20 für eine langsame Freisetzung des Implantats 105 beschrieben. Dies ist beispielsweise der Fall, wenn der Bediener erwartet, dass der Katheter richtig positioniert ist und "am Platz bleibt". Somit testet er mit einer vorsichtigen Freisetzung die Positionierung des Implantats 105. Hierbei wird nun das zweite Griffsegment 20 in Umfangsrichtung 22 gedreht, dabei läuft das Koppelelement 26 bzw. die Kugel 58 wegen des Wirk- bzw. Kraftschlusses auf dem axial fixierten Gewinde 24 mit. Hierdurch wird das Koppelelement 26 und das Übertragungselement 38 axial und je nach Gewindeorientierung und Drehrichtung in Richtung des proximalen oder distalen Endes 12, 14 bewegt. Durch den Formschluss des Übertragungselements 38 mit dem Verbindungselement 30 wird auch diese zusammen mit dem äußeren Einführelement 94 axial bewegt. Hierbei wird die Drehbewegung nicht auf das Verbindungselement 30 übertragen, da dieses durch die Fixierelemente 32 in Umfangsrichtung 22 fixiert ist. Die Fixierelemente 32 ermöglichen in Zusammenwirkung mit den Ausnehmungen 36 auch eine axiale Führung der Bewegung. Zudem gewährleisten die Fixierelemente 32 auch über den Eingriff in die Vertiefungen 56 des Grundkörpers 44 eine in Umfangsrichtung 22 und axiale Richtung 46 fixierte Anordnung des Gewindes 24. Letztendlich entsteht durch Drehen des zweiten Griffsegments 20 über das Kugelumlaufgewinde des Grundkörpers 44 eine axiale Bewegung, die das äußeren Einführelement 94 relativ zu dem inneren Einführelement 92 und der Außenhülle 96 verschiebt.

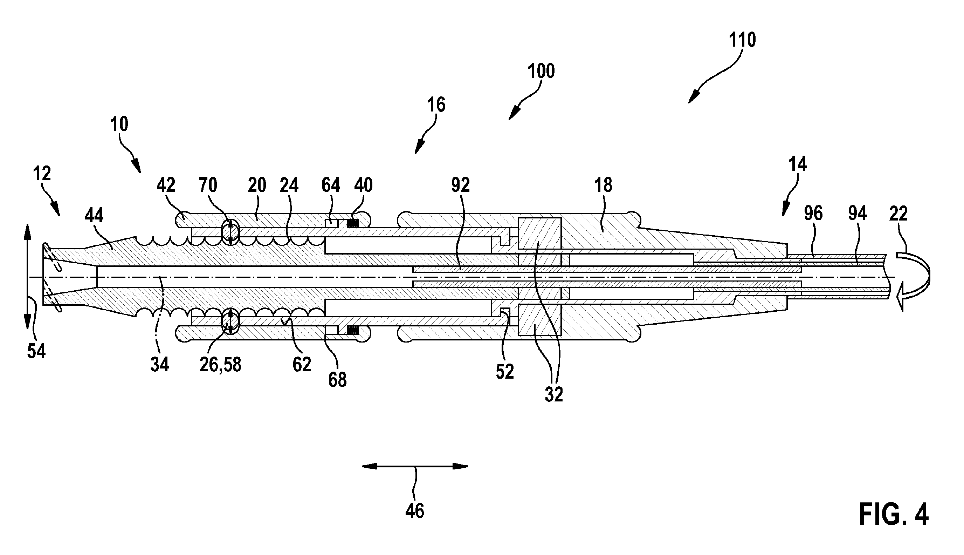

Die Freigabevorrichtung 100 weist zusätzlich einen Mechanismus für eine schnelle Freigabe des Implantats 105 auf, wie dies in Fig. 4 dargestellt ist. Hierbei ist der Wirkschluss zwischen dem zweiten Griffsegment 20 und dem Gewinde 24 durch eine Bewegung eines Entriegelungselements 42 gegen die Federspannung der Federelemente 40 auflösbar. Das Entriegelungselement 42 ist einstückig mit dem zweiten Griffsegment 20 ausgebildet bzw. von diesem gebildet. Für die schnelle Freisetzung wird nun beispielsweise das zweite Griffsegment 20 in Richtung des proximalen Endes 12 des Körpers 10 gezogen, was für einen Bediener intuitiv erscheint, da hierbei das äußere Einführelement 94 vom Implantat 105 "abgezogen" wird. Durch diese Bewegung des Entriegelungselements 42 bzw. des zweiten Griffsegments 20 wird das Koppelelement 26 radial weg von der Innenachse 34 der Einführvorrichtung 110 bewegt und kommt dadurch außer Eingriff mit dem Gewinde 24. Dies findet statt, da das Koppelelement 26 in eine Aufnahme 70 ausweichen kann, die an der Innenseite 62 des zweiten Griffsegments 20 ausgeformt ist. Somit kann ein axialer Zug jederzeit auf das äußere Einführelement 94 übertragen werden, ohne dass ein Betriebszustand im Vorfeld aufwendig umgeschaltet oder ein Bediener die Handgriffeinheit 16 umgreifen muss, da die für das äußere Einführelement 94 zuständige Hand bereits am zweiten Griffsegment 20 angreift.

Zu einer Implantation des Implantats 105 im Körper, wird in einem ersten Schritt die Einführvorrichtung 110 bzw. die Einführelemente 92, 94 zu deren Entlüftung gespült. Hierfür wird eine nicht gezeigte Spritze an das Luer-Lock des Grundkörpers 44 angeschlossen und es wird ein Spülmedium, wie beispielsweise physiologische Kochsalzlösung, über die Spritze in das innere Einführelement 92 und über Öffnungen in diesem in das äußere Einführelement 94 eingebracht. Die so vorbereitete mit dem Implantat 105 versehene Einführvorrichtung 110 wird nun in den Körper eingeführt. Nach einer ersten Platzierung des distalen Endes 120 der Einführvorrichtung 110 am Implantationsort wird das Implantat 105 mittels der Drehung des zweiten Griffsegments 20 langsam freigesetzt. Hierdurch wird erst das distale Ende 109 des Implantats 105 freigegeben, das sich wegen der Ausführung des Implantats 105 als selbstexpandierendes Implantat, wie beispielsweise eine Stent, selbsttätig expandiert. Ist eine exakte Positionierung des Implantats 105 erreicht, was durch ein Überwachungsgerät wie ein Röntgengerät, beobachtet werden kann, wird mittels der schnellen Freisetzung das gesamte Implantat 105 bzw. ein proximales Ende 107 freigegeben und aufgrund seiner Radialkraft vollständig geöffnet. Im Anschluss wird die Freigabevorrichtung 100 mit dem Innenschaft in den Außenschaft zurückgezogen und die Einführvorrichtung 110 aus dem Körper entfernt. Das Implantat 105 verbleibt vollständig positioniert im Körper (nicht gezeigt).

Grundsätzlich kann sowohl der Mechanismus der langsamen wie auch der der schnellen Freisetzung zu einer Montage des Implantats 105 auf der Einführvorrichtung 110 genutzt werden. Dies ist vor allem möglich, da wegen der "Untersetzung" der Bewegung hohe Kräfte realisiert werden können. Hierbei wird das Implantat 105 auf einen freigelegten Bereich des inneren Einführelements 92 am distalen Ende 120 der Einführvorrichtung 110 befestigt, beispielsweise mittels eines Crimpprozesses. Im Anschluss wird das Implantat 105 von äußeren Einführelement 94 abgedeckt. Hierfür wird das zweite Griffsegment 20 entweder in die entgegengesetzte Richtung wie bei der Freigabe gedreht oder in Richtung des distalen Endes 14 des Körpers 10 gedrückt. Dabei wird aus einer Position des zweiten Griffelements 20 gestartet, bei der dieses in der proximal hinteren, schnellen Freigabeposition ist. Um hier vor der Befestigung des Implantats 105 am inneren Einführelement 92 eine vorschnelle axiale Bewegung, vermittelt durch das in dieser Position gestauchte Federelement 40, zu verhindern, kann ein hier nicht näher gezeigtes Verriegelungselement am zweiten Griffsegment 20 vorgesehen sein. Die Druckbewegung wird hier durch das gestauchte Federelement 40 unterstützt. Nach dem Abdecken des Implantats 105 durch das äußere Einführelement 94 ist die Montage abgeschlossen.

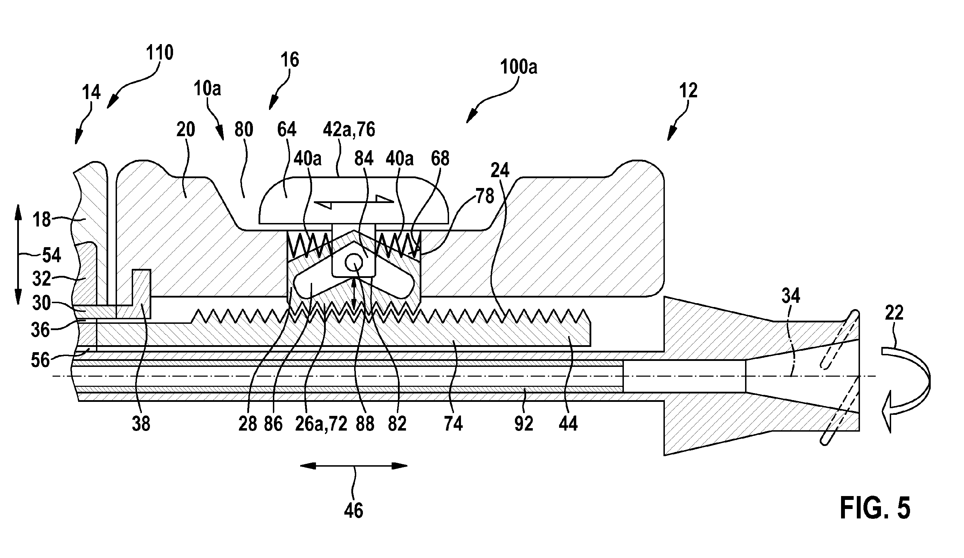

In Fig. 5 ist ein alternatives Ausführungsbeispiel des Körpers 10 und der Freigabevorrichtung 100 dargestellt. Im Wesentlichen sind gleich bleibende Bauteile, Merkmale und Funktionen grundsätzlich mit den gleichen Bezugszeichen beziffert. Zur Unterscheidung des Ausführungsbeispiels der Fig. 5 zu dem in den Fig. 1 bis 4 ist jedoch den Bezugszeichen der in dem Ausführungsbeispiel der Fig. 5 abweichenden ausgeführten Bauteile der Buchstabe a hinzugefügt. Die nachfolgende Beschreibung beschränkt sich im Wesentlichen auf die Unterschiede zu dem Ausführungsbeispiel in den Fig. 1 bis 4, wobei bezüglich gleich bleibender Bauteile, Merkmale und Funktionen auf die Beschreibung des Ausführungsbeispiels in den Fig. 1 bis 4 verwiesen werden kann.

Der Körper 10a bzw. die Freigabevorrichtung 100a der Fig. 5 unterscheidet sich von dem Körper 10 der Fig. 1 bis 4 dadurch, dass der Körper 10a ein alternativ ausgeführtes Koppelelement 26a aufweist. Das Koppelelement 26a ist von einem Verzahnungselement 28, in der Form einer Zahnstange 72, gebildet. Somit erfolgt der Wirkschluss, der hier ein Formschluss ist, zwischen dem zweiten Griffsegment 20a und einem Gewinde 24 über das Verzahnungselement 28. Das Gewinde 24 selbst kann hierbei eine Spindel 47, ausgebildet an einem Grundkörper 44a, sein.

Zudem unterscheidet sich der Körper 10a der Fig. 5 dadurch, dass das Entriegelungselement 42a alternativ ausgebildet ist. Das Entriegelungselement 42a ist separat von dem zweiten Griffsegment 20a ausgebildet und weist als mechanisches Bedienelement einen sich in Umfangsrichtung 22 um das zweite Griffsegment 20a erstreckenden Ring 76 auf. Das Verzahnungselement 28 ist radial zwischen dem Entriegelungselement 42a und dem Grundkörper 44a in einer Durchgangsöffnung 78 des zweiten Griffsegments 20a angeordnet. Der Ring 76 ist zudem in einer radialen Einbuchtung 80 des zweiten Griffsegments 20a axial verschieblich angeordnet. Für eine schnelle Freigabe eines hier nicht gezeigten Implantats wird das Entriegelungselement 42a bzw. der Ring 76 in Richtung eines proximalen Endes 12 des Körpers 10a verschoben. Die Bewegung des Entriegelungselements 42a erfolgt hier gegen eine Federspannung eines Federelements 40a, das das Entriegelungselement 42a in seinem axialen Ruhezustand vorspannt. Das Federelement 40a ist von einer Druckfeder gebildet, die axial zwischen einer Wandung 68 der Durchgangsöffnung 78 des zweiten Griffsegments 20a und einem sich in radialer Richtung 54 zur Innenachse 34 erstreckenden Fortsatz 82 des Entriegelungselements 42a eingespannt ist. Wird nun das Entriegelungselement 42a axial bewegt läuft ein am Fortsatz 82 angeformter Zapfen 84 entlang einer Kulisse 86. Diese Kulisse 86 verläuft, ausgehend von einem Anlenkpunkt 88 des Zapfens 84 am Koppelelement 26a, schräg zur Innenachse 34 hin. Durch diese Bewegung des Zapfens 84 wird das Koppelelement 26a radial weg von einer Innenachse 34 der Einführvorrichtung 110 bewegt und kommt dadurch außer Eingriff mit dem Gewinde 24, wodurch der Wirkschluss zwischen dem zweiten Griffsegment 20a und dem Gewinde 24 auflösbar ist. Hierbei wird eine homogene Bewegung des Koppelelements 26a durch dessen Führung mittels der Wandungen 68a der Durchgangsöffnung 78 erreicht. Die Relativbewegung zwischen dem inneren und äußeren Einführelement 92, 94 wird nun letztendlich dadurch bewirken, dass das zweite Griffsegment 20a nach der Entriegelung durch den Bediener in Richtung des proximalen Endes 12 des Körpers 10a gezogen wird.

### Bezugszeichen

- 10: Körper
- 12: Ende
- 14: Ende
- 16: Handgriffeinheit
- 18: Griffsegment
- 20: Griffsegment
- 22: Umfangsrichtung
- 24: Gewinde
- 26: Koppelelement
- 28: Verzahnungselement
- 30: Verbindungselement
- 32: Fixierelement
- 34: Innenachse
- 36: Aussparung
- 38: Übertragungselement
- 40: Federelement
- 42: Entriegelungselement
- 44: Grundkörper
- 46: axiale Richtung
- 48: Durchführung
- 50: Fortsatz
- 52: Nut
- 54: radiale Richtung
- 56: Vertiefung
- 58: Kugel
- 60: Kugelkäfig
- 62: Innenseite
- 64: Aussparung
- 66: Fortsatz
- 68: Wandung
- 70: Aufnahme
- 72: Zahnstange
- 74: Spindel
- 76: Ring
- 78: Durchgangsöffnung
- 80: Einbuchtung
- 82: Fortsatz
- 84: Zapfen
- 86: Kulisse
- 88: Anlenkpunkt
- 90: Schaftbereich
- 92: Einführelement
- 94: Einführelement
- 96: Außenhülle
- 100: Freigabevorrichtung
- 105: Implantat
- 107: Ende
- 109: Ende
- 110: Einführvorrichtung
- 115: Ende
- 120: Ende
- 125: Katheterspitze

## Patentansprüche

1. Freigabevorrichtung (100, 100a) zum Lösen eines medizinisches Implantats (105) von einer Einführvorrichtung (110), bei welcher das Implantat (105) durch eine Relativbewegung zwischen einem ersten und einem zweiten Einführelement (92, 94) freisetzbar ist, umfassend einen Körper (10, 10a) mit einem proximalen Ende (12), mit einem proximalen Ende (12), das im Benutzungszustand von einem distalen Ende (120) der Einführvorrichtung (110) entfernt ist, und einem distalen Ende (14), das im Benutzungszustand dem distalen Ende (120) der Einführvorrichtung (110) zugewandt ist, wobei zwischen dem proximalen und dem distalen Ende (12, 14) eine Handgriffeinheit (16) mit einem relativ zum Körper (10, 10a) fixierten ersten Griffsegment (18) und zumindest einem beweglich angeordneten zweiten Griffsegment (20, 20a) vorgesehen ist, wobei das beweglich angeordnete zweite Griffsegment (20, 20a) in Umfangsrichtung (22) drehbar ist, um einer gezielte Relativbewegung zwischen dem ersten und dem zweiten Einführelement (92, 94) der Einführvorrichtung (110) zu bewirken.

2. Freigabevorrichtung nach Anspruch 1, wobei das zweite Griffsegment (20, 20a) die Relativbewegung zwischen dem ersten und dem zweiten Einführelement (92, 94) der Einführvorrichtung (110) über zumindest einen Wirkschluss mit einem Gewinde (24) vermittelt.

3. Freigabevorrichtung nach Anspruch 1 oder 2, wobei der Wirkschluss zwischen dem zweiten Griffsegment (20, 20a) und dem Gewinde (24) über zumindest ein Koppelelement (26, 26a) erfolgt.

4. Freigabevorrichtung nach einem der vorhergehenden Ansprüche, wobei der Wirkschluss zwischen dem zweiten Griffsegment (20) und dem Gewinde (24) zumindest einen Kraftschluss darstellt.

5. Freigabevorrichtung nach einem der vorhergehenden Ansprüche, wobei der Wirkschluss zwischen dem zweiten Griffsegment (20a) und dem Gewinde (24) über zumindest ein Verzahnungselement (28) erfolgt.

6. Freigabevorrichtung nach einem der vorhergehenden Ansprüche, wobei die Relativbewegung zwischen dem ersten und dem zweiten Einführelement (92, 94) der Einführvorrichtung (110) mittels eines Verbindungselements (30) auf eines der Einführelemente (94) übertragbar ist, wobei das Verbindungselement (30) wirkmäßig mit dem zweiten Griffsegment (20, 20a) verbunden ist.

7. Freigabevorrichtung nach einem der vorhergehenden Ansprüche, wobei eine Fixierung des Gewindes (24) mittels zumindest eines Fixierelements (32) erfolgt, das sich im Wesentlichen radial zu einer Innenachse (34) der Einführvorrichtung (110) erstreckt.

8. Freigabevorrichtung zumindest nach Anspruch 6 und 7, wobei das Verbindungselement (30) zumindest eine Ausnehmung (36) aufweist, in die das zumindest eine Fixierelement (32) eingreift, um das Verbindungselement (30) in Umfangsrichtung (22) zu fixieren.

9. Freigabevorrichtung zumindest nach Anspruch 6, aufweisend ein Übertragungselement (38), das über einen Formschluss mit dem Verbindungselement (30) verbunden ist, wobei das Übertragungselement (38) relativ zum Verbindungselement (30) drehbar angeordnet ist.

10. Freigabevorrichtung nach einem der vorhergehenden Ansprüche, wobei das zweite Griffsegment (20) in seinem axialen Ruhezustand durch zumindest ein Federelement (40) vorgespannt ist.

11. Freigabevorrichtung zumindest nach Anspruch 2, wobei der Wirkschluss zwischen dem zweiten Griffsegment (20, 20a) und dem Gewinde (24) durch eine Bewegung zumindest eines Entriegelungselements (42, 42a) gegen eine Federspannung zumindest eines Federelements (40, 40a) auflösbar ist.

12. Freigabevorrichtung zumindest nach Anspruch 3 und 11, wobei sich durch die Bewegung des zumindest einen Entriegelungselements (42, 42a) das zumindest eine Koppelelement (26, 26a) radial weg von einer Innenachse (34) der Einführvorrichtung (110) bewegt und dadurch außer Eingriff mit dem Gewinde (24) kommt.

13. Freigabevorrichtung nach Anspruch 11 oder 12, wobei das zumindest eine Entriegelungselement (42) einstückig mit dem zweiten Griffsegment (20) ausgebildet ist.

14. Einführvorrichtung (110) zum Einführen eines medizinischen Implantats (105), welches durch eine Relativbewegung zwischen einem ersten und einem zweiten Einführelement (92, 94) freisetzbar ist, umfassend eine Freigabevorrichtung (100, 100a) zum Lösen des medizinisches Implantats (105), insbesondere nach einem der Ansprüche 1 bis 13, umfassend einen Körper (10, 10a) mit einem proximalen Ende (12), das im Benutzungszustand von einem distalen Ende (120) der Einführvorrichtung (110) entfernt ist, und einem distalen Ende (14), das im Benutzungszustand dem distalen Ende (120) der Einführvorrichtung (110) zugewandt ist, wobei zwischen dem proximalen und dem distalen Ende (12, 14) eine Handgriffeinheit mit einem relativ zum Körper (10, 10a) fixierten ersten Griffsegment (18) und zumindest einem beweglich angeordneten zweiten Griffsegment (20, 20a) vorgesehen ist, wobei das beweglich angeordnete zweite Griffsegment (20, 20a) in Umfangsrichtung (22) drehbar ist, um einer gezielte Relativbewegung zwischen dem ersten und dem zweiten Einführelement (92, 94) der Einführvorrichtung (110) zu bewirken.
